# EUROPEAN PATENT SPECIFICATION

(11) **EP 4 236 794 B1**
(45) Date of publication and mention of the grant of the patent: **27.05.2026**
(21) Application number: 21823395.5
(22) Date of filing: 26.10.2021
(51) Int. Cl.: A61B 5/145

(54) **ROLLED MULTILAYER CHEMICAL SENSING ELEMENTS AND DEVICES AND SYSTEM INCLUDING THE SAME**
GEROLLTE MEHRSCHICHTIGE CHEMISCHE SENSORELEMENTE UND VORRICHTUNGEN UND SYSTEM DAMIT
ÉLÉMENTS DE DÉTECTION CHIMIQUE MULTICOUCHE ENROULÉS, AINSI QUE DISPOSITIFS ET SYSTÈME COMPRENANT CEUX-CI

(30) Priority: 29.10.2020 US 202063107347 P
(43) Date of publication of application: 06.09.2023
(73) Proprietor: Cardiac Pacemakers, Inc., St. Paul, MN 55112-5798 (US)
(72) Inventor: LI, Yingbo, Woodbury, Minnesota 55129 (US); KANE, Michael J., St. Paul, Minnesota 55105 (US); WULFMAN, David Robert, Minneapolis, Minnesota 55405 (US); AREMU, Adeniyi O., Ramsey, Minnesota 55303 (US)
(74) Representative: Pfenning, Meinig & Partner mbB
(86) International application number: PCT/US2021/056590
(87) International publication number: WO 2022/093777

(56) References cited:
- EP-A1- 3 409 203
- WO-A1-2017/027782
- US-A1- 2019 059 792

## Description

### Field

Embodiments herein relate to chemical sensing elements and, more specifically, to chemical sensing elements including a rolled multilayer structure.

### Background

Data regarding physiological analytes are highly relevant for the diagnosis and treatment of many conditions and disease states. As one example, potassium ion concentrations can affect a patient's cardiac rhythm. Therefore, medical professionals frequently evaluate physiological potassium ion concentration when diagnosing a cardiac rhythm problem. However, measuring physiological concentrations of analytes, such as potassium, generally requires drawing blood from the patient. Blood draws are commonly done at a medical clinic or hospital and therefore generally require the patient to physically visit a medical facility. As a result, despite their significance, physiological analyte concentrations are frequently measured only sporadically during the odyssey of medical treatment of a given patient.

Implantable chemical sensors can be used to gather data about physiological analytes while a patient is away from a medical care facility and without needing to draw blood or another fluid from the patient. However, the design and construction of effective chemical sensors is subject to many challenges. EP3409203A1 relates to implantable chemical sensors for detecting a physiological analyte and medical devices including the same.

### Summary

The invention is defined in claims 1 and 7. Embodiments herein relate to chemical sensing elements including a rolled multilayer structure.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense.

### Brief Description of the Figures

Aspects may be more completely understood in connection with the following figures (FIGS.), in which:
FIG. 1 is a schematic top view of an implantable medical device in accordance with various embodiments herein.
FIG. 2 is a schematic view of an electrospinning process in accordance with various embodiments herein.
FIG. 3 is a schematic view of a mandrel coated with a fibrous layer in accordance with various embodiments herein.
FIG. 4 is a cross-sectional view of a mandrel coated with a fibrous layer taken along line 4-4' of FIG. 3.
FIG. 5 is a schematic view of a chemical sensor composition infused fibrous coated mandrel in accordance with various embodiments herein.
FIG. 6 is a cross-sectional view a chemical sensor composition infused fibrous coated mandrel taken along line 6-6' of FIG. 5.
FIG. 7 is a schematic view of a hydrogel coated mandrel in accordance with various embodiments herein.
FIG. 8 is a cross-sectional view of a hydrogel coated mandrel taken along line 8-8' of FIG. 7.
FIG. 9 is a cross-sectional view of a rolled multilayer film on a mandrel in accordance with various embodiments herein.
FIG. 10 is a schematic view of hydrogel being sprayed onto a chemical sensor element in accordance with various embodiments herein.
FIG. 11 is a schematic view of a chemical sensor element in accordance with various embodiments herein.
FIG. 12 is a schematic view of a chemical sensor element in accordance with various embodiments herein.
FIG. 13 is a schematic top view of a chemical sensor element disposed in a well in accordance with various embodiments herein.
FIG. 14 is a schematic top view of chemical sensor elements disposed in a well in accordance with various embodiments herein.
FIG. 15 is a flow diagram a method of making a chemical sensor element in accordance with various embodiments herein.
FIG. 16 is a flow diagram a method of making a chemical sensor element in accordance with various embodiments herein.
FIG. 17 is a schematic view of an implantable medical device in accordance with various embodiments herein.
FIG. 18 is a schematic diagram of components of an implantable medical device in accordance with various embodiments herein.
FIG. 19 is a schematic view of a fibrous layer being sprayed onto a mandrel in accordance with various embodiments herein.
FIG. 20 is a schematic view of a deposition substrate in accordance with various embodiments herein.
FIG. 21 is a schematic view of a chemical sensor element including a light pipe in accordance with various embodiments herein.
FIG. 22 is a cross-sectional view of a chemical sensor element including a light pipe taken along line 22-22' of FIG. 21.
FIG. 23 is a schematic view of a chemical sensor element being rolled around a light pipe in accordance with various embodiments herein.

While embodiments are susceptible to various modifications and alternative forms, specifics thereof have been shown by way of example and drawings, and will be described in detail. It should be understood, however, that the scope herein is not limited to the particular aspects described. On the contrary, the intention is to cover modifications, and alternatives falling within the scope herein.

### Detailed Description

Physicians frequently analyze a patient's chemical analyte concentrations when assessing a patient's condition and/or diagnosing a patient's medical problem. For example, electrolyte concentrations (specifically including potassium) are commonly measured and analyzed by physicians due their impact on many bodily systems including the heart. Traditionally, chemical analyte concentrations are assessed by drawing a fluid sample, such as blood, from the patient while at a medical clinic or hospital. However, then a patient's chemical analyte concentrations can only be monitored when a patient physically visits a medical clinic or hospital.

Implantable analyte sensors allow for chemical analyte concentrations to be measured in real time even when a patient is away from a medical clinic or hospital and without the need to draw any fluids from the patient. This allows for a patient's chemical analyte concentrations to be monitored more frequently, for example continuously or semi-continuously, and allow physicians to gather larger data sets to be evaluated.

However, implantable analyte sensors require rapid diffusion and high permeability to be effective. While some implanted analyte sensors may allow for rapid diffusion and high permeability their manufacture is cumbersome. Embodiments herein, however, can include an implantable analyte sensor using multiple layers and a rolled construction to achieve rapid diffusion and high permeability while also being readily manufactured. Also, when a rolled construction is oriented with the end toward a source of chemical analytes the hydrophilic or hydrogel portion can serve as an ionic conduit facilitating diffusion of chemical analytes.

In some embodiments, one or more layers can be fabricated using an electrospinning process. Electrospinning allows the implantable analyte sensor to have high interaction areas, high permeability, and non-periodic structures amenable to photo interrogation.

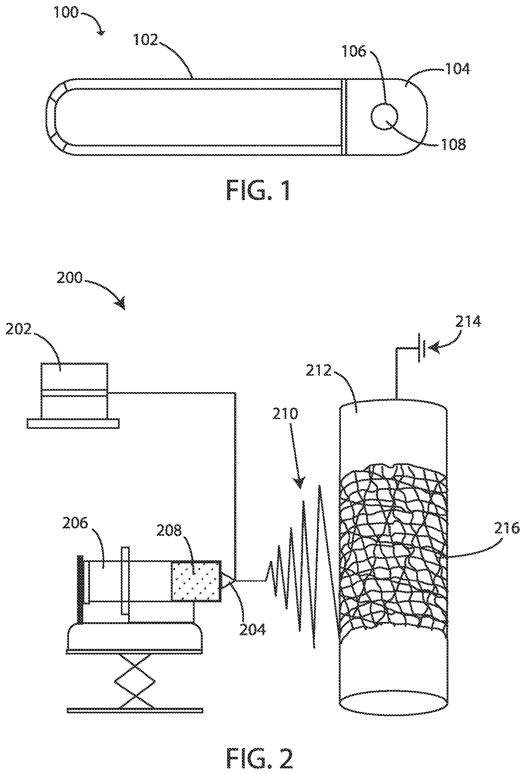

Referring now to FIG. 1 an implantable medical device 100 is shown in accordance with various embodiments herein. The implantable medical device 100 can include an implantable housing 102 and a header 104 coupled to the implantable housing 102. Various materials can be used. However, in some embodiments, the implantable housing 102 can be formed of a material such as a metal, ceramic, a polymer, or a composite. The header 104 can be formed of various materials, but in some embodiments the header 104 can be formed of a polymer (translucent or opaque) such as an epoxy material. In some embodiments the header 104 can be hollow. In other embodiments the header 104 can be filled with components and/or structural materials such as epoxy or another material such that it is non-hollow. In some embodiments, however, a distinct header 104 can be omitted. Rather, the implantable housing 102 can include substantially all of the components of the device.

The implantable medical device 100 can also include a well 106 coupled to the implantable housing 102 configured to hold components of a chemical sensor 108 such as one or more chemical sensor elements (not shown in this view). The chemical sensor element(s) can be configured to detect an ion concentration of a bodily fluid when implanted in the body. Bodily fluids can include blood, interstitial fluid, serum, lymph, serous fluid, cerebrospinal fluid, and the like. In some embodiments the chemical sensor element can be configured to detect one or more of an electrolyte, a protein, a sugar, a hormone, a peptide, an amino acid and a metabolic product. In some embodiments, the chemical sensor element can be configured to detect an ion selected from a group consisting of potassium, sodium, chloride, calcium, magnesium, lithium, hydronium, hydrogen phosphate, bicarbonate, and the like. However, many other chemical analytes are also contemplated herein.

It will be appreciated that the well 106 can be positioned at any location along implantable medical device 100, including along the implantable housing 102 or along the header 104. It will also be appreciated that though FIG. 1 shows a device having one well 106, any number of wells can be present. For example, the device can include at least 1, 2, 3, 4, 5, 6, 7, 8, 9, 10, 11, 12, or more wells, or a number of wells falling within a range between any of the foregoing.

The implantable medical device 100 can take on various dimensions. In some embodiments herein it can be approximately 2 to 3 inches in length, 0.4 to 0.6 inches wide, and 0.15 to 0.35 inches thick. However, in some embodiments, the implantable medical device 100 can be about 0.25, 0.50, 1.0, 2.0, 3.0, 4.0, or 5.0 inches in length. In some embodiments the length can be in a range wherein any of the foregoing lengths can serve as the upper or lower bound of the range, provided that the upper bound is greater than the lower bound. In some embodiments, the implantable medical device 100 can be about 0.25, 0.50, 0.75, 1.0, or 2.0 inches in width. In some embodiments the length can be in a range wherein any of the foregoing lengths can serve as the upper or lower bound of the range, provided that the upper bound is greater than the lower bound. In some embodiments, the implantable medical device 100 can be about 0.10, 0.25, 0.50, 0.75, or 1.0 inches thick. In some embodiments the thickness can be in range wherein any of the foregoing thickness can serve as the upper or lower bound of the range, provided that the upper bound is greater than the lower bound.

In some embodiments, an electrospinning process can be used, in part, to create a chemical sensor element (described in detail below). Referring now to FIG. 2, an electrospinning process 200 is shown in accordance with various embodiments herein. The electrospinning process 200 can be used to create a fibrous layer (described in detail below) of the chemical sensor element. FIG. 2 shows a power supply 202 that provides the power to produce an electric field between a polymer composition 208 at a tip 204 of a syringe 206 and a deposition substrate.

The deposition substrate can include a mandrel 212 that is grounded 214. The electric field created between the tip 204 and the mandrel 212 creates an electrostatic force that causes a surface tension of the droplet of the polymer composition 208 to be overcome. When the surface tension of the droplet of the polymer composition 208 is overcome by the electrostatic forces created, the droplet of the polymer composition 208 becomes a charged, continuous jet of electrospun fibers 210 that rapidly dry and thin in the air as the electrospun fibers 210 move toward the mandrel 212. The electrospun fibers 210 are deposited on the mandrel 212 as deposited fibers 216. In some embodiments, the deposited fibers 216 are arranged in a nonwoven, random orientation.

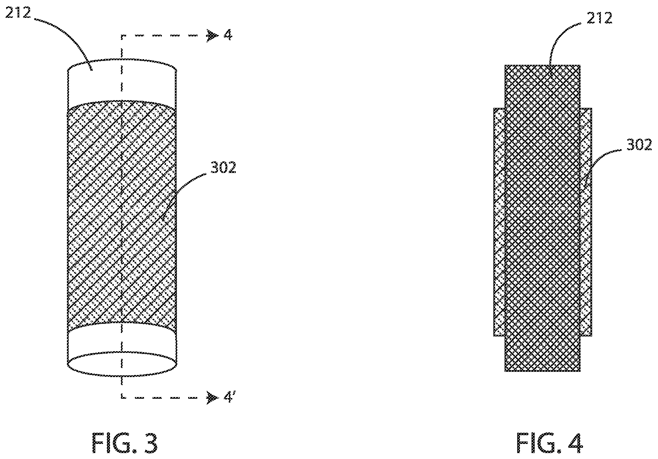

While a variety of techniques can be used, in some embodiments, the electrospinning process 200 can be used, in part, to create a chemical sensor element held in the well 106 of the implantable medical device 100. Referring now to FIG. 3, a mandrel coated with a fibrous layer is shown in accordance with various embodiments herein. The electrospinning process 200, as discussed above, can be used to deposit a polymer layer onto the mandrel 212. In some embodiments, the deposited polymer layer can specifically be a hydrophobic polymer layer. The hydrophobic polymer layer can be formed, in full or in part, from a biocompatible thermoplastic polymer. Exemplary materials for the hydrophobic polymer layer are described in greater detail below.

The hydrophobic polymer layer can be a fibrous layer 302. The fibrous layer 302 can form a first layer of a chemical sensor element that includes a rolled multilayer film. In various embodiments, the fibrous layer 302 can extend for at least 3 or more revolutions around a center of the rolled multilayer film. It will also be appreciated that the fibrous layer 302 can extend for fewer or greater revolutions. For example, the fibrous layer 302 can extend for at least 1, 2, 3, 4, 5, 6, or more revolutions around the center of the rolled multilayer film, or a number of revolutions falling within a range between any of the foregoing.

Referring now to FIG. 4, a cross-sectional view of a mandrel coated with a fibrous layer as taken along line 4-4' of FIG. 3 is shown in accordance with various embodiments herein. The fibrous layer 302 deposited on mandrel 212 can be of varying thicknesses. It is noted the thickness of the fibrous layer 302 should be sufficiently thin enough to be able to be rolled. However, the fibrous layer should also be sufficiently thick that it can hold a sufficient amount of sensing composition that is later infused into the fibrous layer to allow for an analyte of interest to be detected. That being said, it will be appreciated that various components can be deposited with the fibrous layer simultaneously versus later being infused.

In some embodiments herein the fibrous layer 302 can be about 0.5, 0.75, 1, 2, 5, 10, 20, 50, 100, 200, 500 microns or more in thickness. In some embodiments the thickness can fall within a range wherein any of the foregoing thicknesses can serve as the upper or lower bound of the range, provided that the upper bound is greater than the lower bound.

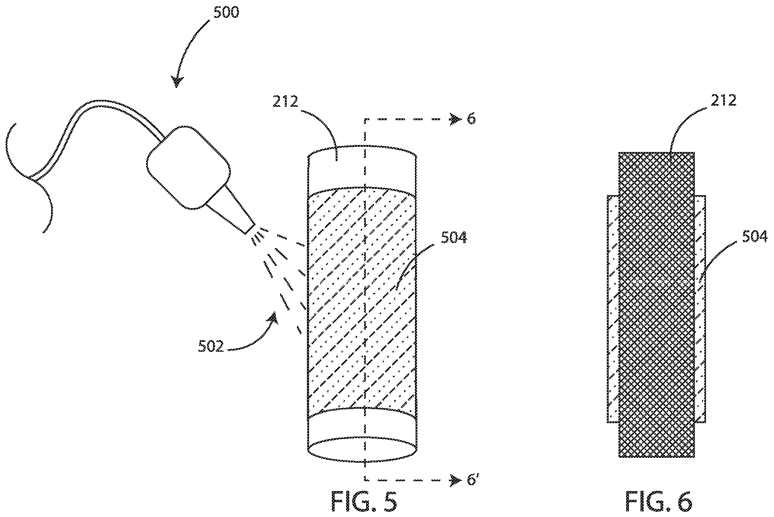

In some embodiments, the fibrous layer 302 (or the hydrophobic layer) can be infused with a chemical sensor composition. Referring now to FIG. 5, a chemical sensor composition infused fibrous layer coated mandrel is shown in accordance with various embodiments herein. The fibrous layer 302 (discussed above) can be infused with a chemical sensing composition 502 to form a fibrous layer with infused sensing composition 504. In some embodiments, the chemical sensing composition 502 can be sprayed on the fibrous layer 302 using any conventional spraying device such as sprayer 500. However, it will be appreciated that the chemical sensing composition 502 can also be applied using other techniques including, but not limited to, dip coating, blade coating, printing deposition, and the like.

After being sprayed on (or otherwise applied), the chemical sensing composition 502 can be absorbed/infused into the fibrous layer 302. However, it will be appreciated that the chemical sensing composition 502 can also be sprayed on (or otherwise applied) together with the fibrous layer 302 simultaneously. The chemical sensing composition 502 can effectively be dispersed within the fibrous layer 302. In some embodiments, the chemical sensing composition 502 can be formed, in full or in part, from a lipophilic indicator dye, such as a lipophilic fluorescent indicator dye, a lipophilic colorimetric indicator dye, or the like. Exemplary materials for the chemical sensing composition 502 are described in greater detail below. Referring now to FIG. 6, a cross-sectional view of a chemical sensor composition infused fibrous layer coated mandrel as taken along line 6-6' of FIG. 5 is shown in accordance with various embodiments herein.

In some embodiments, the fibrous layer that is infused with a sensing composition 504 can be coated with a hydrophilic polymer layer. The hydrophilic polymer layer can be formed, in full or in part, from a permeable material, such as an ion permeable polymeric matrix material. Exemplary materials for the hydrophilic polymer layer are described in greater detail below. However, in various embodiments the hydrophilic polymer layer can include a hydrogel layer permeable to a variety of ions for example potassium ions, sodium ions, chloride ions, and the like or other analytes.

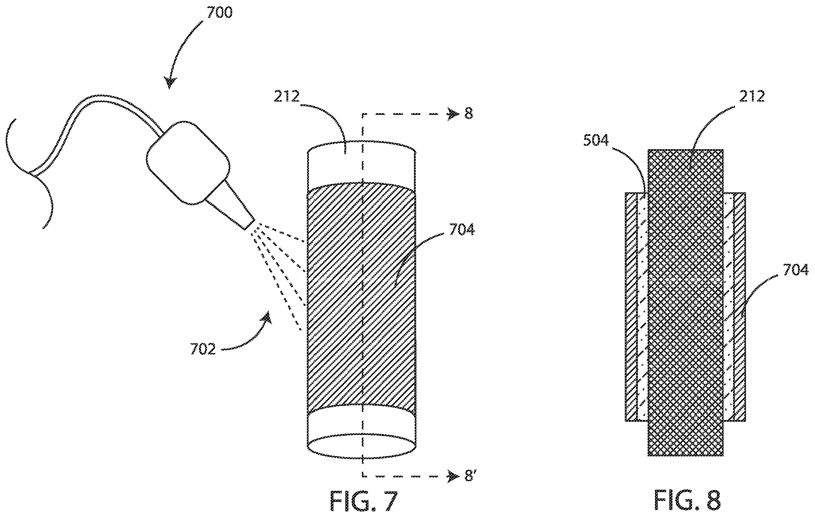

Referring now to FIG. 7 a mandrel is shown as coated with a fibrous layer that is infused with a sensing composition and further coated with a hydrophilic polymer layer in accordance with various embodiments herein. In this example, the hydrophilic polymer layer is a hydrogel layer 704. The hydrogel layer 704 can form a second layer of the chemical sensor element. In some embodiments, a hydrogel composition 702 can be sprayed onto the underlying fibrous layer to form the hydrogel layer 704. The hydrogel composition 702 can be sprayed on using any conventional spraying device such as sprayer 700. However, it will be appreciated that the hydrogel layer 704 can also be applied using other techniques including, but not limited to, dip coating, blade coating, printing deposition, and the like. In some embodiments, the hydrogel layer 704 can be formed with *in-situ* polymerization.

When the materials disposed on the mandrel are later rolled (as described below), the hydrogel layer 704 ends up separating adjacent layers of the fibrous layer that is infused with a sensing composition. In this way, the hydrogel layer 704 acts to retain the sensing composition within the fibrous layer.

Referring now to FIG. 8, a cross-sectional view of a hydrogel coated mandrel taken along line 8-8' of FIG. 7 is shown in accordance with various embodiments herein. The fibrous layer with infused sensing composition 504 can form a first layer of a chemical sensor element and a hydrogel layer 704 can form a second layer of the chemical sensor element. The hydrogel layer 704 can have varying thickness. The thickness of the hydrogel layer 704 should be sufficiently thin so as to be able to be rolled. However, the thickness of the hydrogel layer 704 should be sufficiently thick so as to effectively prevent outmigration of the sensing composition from the fibrous layer.

In various embodiments the hydrogel layer 704 can be about 0.5, 0.75, 1, 2, 5, 10, 20, 50, 100, 200, 500 microns or more in thickness. In some embodiments the thickness can be in range wherein any of the foregoing thickness can serve as the upper or lower bound of the range, provided that the upper bound is greater than the lower bound.

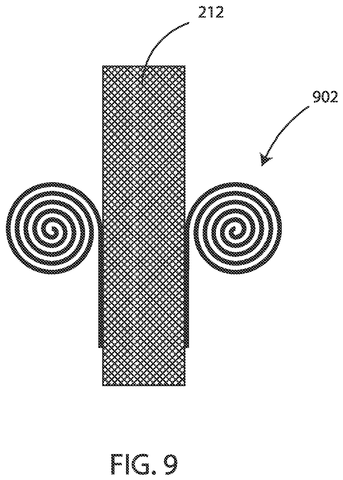

In various embodiments, materials can be removed from the mandrel 212 to form a portion of the chemical sensing element. For example, materials can be rolled off of the mandrel 212 to form a rolled multilayer structure or film. Referring now to FIG. 9, a cross-sectional view of a rolled multilayer film 902 in the process of being rolled off a mandrel 212 is shown in accordance with various embodiments herein. Rolling can begin at one end of the mandrel and then proceed towards the other end of the mandrel.

In various embodiments, the rolled multilayer film 902 can have a diameter of 0.2 millimeters to 8 millimeters. In some embodiments, the rolled multilayer film 902 can be about 0.1, 0.2, 0.3, 0.4, 0.5, 1, 1.5, 2.5, 3, 4, 5, 6, 7, 8, or 9 millimeters in diameter. In some embodiments the diameter can be in range wherein any of the foregoing diameters can serve as the upper or lower bound of the range, provided that the upper bound is greater than the lower bound.

In some embodiments, the materials can be cut/sliced either before or after the process of rolling. Slicing can be performed using any conventional cutting means, for example by using a razor blade. In some embodiments, the fibrous layer with infused sensing composition 504 and the hydrogel layer 704 can be cut one or more times vertically along the mandrel 212, thus creating different strips to be rolled up. In other embodiments, the fibrous layer with infused sensing composition 504 and the hydrogel layer 704 can be cut one or more times horizontally around the mandrel 212, such as to limit the length of materials to be rolled up. In some embodiments, cutting/slicing operations can be performed after rolling has occurred such as to form discrete rolled portions and set the thickness thereof. In various embodiments, the rolled multilayer film 902 has a thickness of 50 microns to 500 microns. In some embodiments, the rolled multilayer film 902 can be about 25, 50, 75, 100, 125, 150, 200, 250, 300, 350, 400, 450, 500, 550, or 600 in thickness. In some embodiments the thickness can be in range wherein any of the foregoing thickness can serve as the upper or lower bound of the range, provided that the upper bound is greater than the lower bound.

In some embodiments, the rolled multilayer film 902 can be coated with a hydrogel composition 702 on one or more of its exterior surfaces. For example, coating of the rolled multilayer film 902 can serve to coat or cover exposed ends of the fibrous layer so as to prevent outmigration of the sensing composition therefrom.

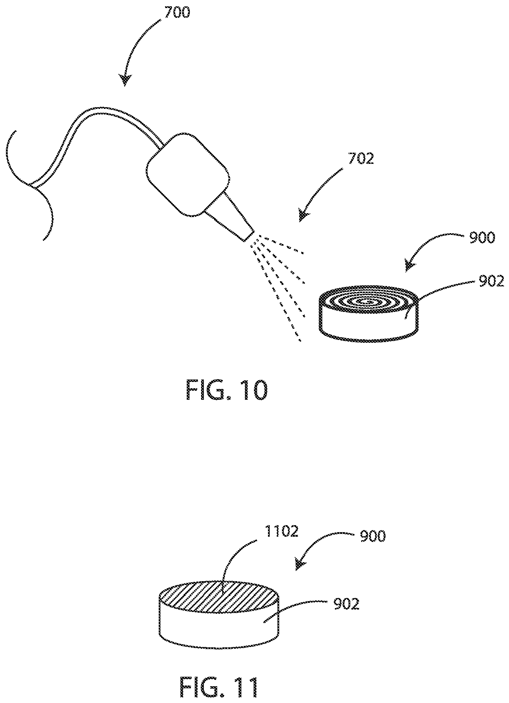

Referring now to FIG. 10, hydrogel composition 702 being sprayed onto a chemical sensor element is shown in accordance with various embodiments herein. FIG. 10 shows a chemical sensor element 900. The chemical sensor element 900 can include the rolled multilayer film 902. The hydrogel composition 702 can be sprayed onto the rolled multilayer film 902. In some embodiments, the hydrogel composition 702 can be sprayed on the rolled multilayer film 902 using any conventional spraying device, for example sprayer 700. However, it will be appreciated that the hydrogel composition 702 can also be applied using other techniques including, but not limited to, dip coating, blade coating, printing deposition, and the like.

Referring now to FIG. 11, a chemical sensor element 900 (or portion thereof) is shown in accordance with various embodiments herein. The chemical sensor element 900 can include a rolled multilayer film 902. The chemical sensor element 900 can also include a top hydrogel layer 1102. The top hydrogel layer 1102 can prevent outmigration of the sensing composition from the otherwise exposed ends of the fibrous layer. While not shown in this view, a similar hydrogel layer can be disposed over the bottom of the chemical sensor element 900.

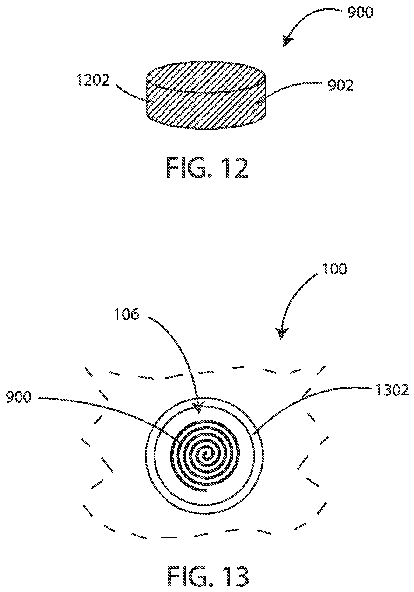

In some embodiments, hydrogel composition 702 (discussed above) can be sprayed on and fully encapsulate the rolled multilayer film 902. Referring now to FIG. 12, a chemical sensor element 900 is shown in accordance with various embodiments herein. The chemical sensor element 900 can include a rolled multilayer film 902 and an encapsulating hydrogel layer 1202. Encapsulating the rolled multilayer film 902 with the hydrogel composition can ensure that there are no outmigration paths for the sensing composition. In some embodiments, the hydrogel layer 1102 can be sprayed on the rolled multilayer film 902 as discussed in FIG. 10. However, other application techniques can also be used including, but not limited to, dip coating, blade coating, printing deposition, and the like. In some embodiments, hydrogel layer 1102 and/or encapsulating hydrogel layer 1202 can be further coated and/or surface modified to further reduce biofouling.

In some embodiments, once formed, the chemical sensor element 900 can be placed in or on implantable medical device 100. Referring now to FIG. 13, chemical sensor element 900 disposed in well 106 is shown in accordance with various embodiments herein. Implantable medical device 100 can include well 106 and a well border 1302 or rim. In some embodiments, the chemical sensor element 900 can be disposed within the well 106. In various embodiments a cover layer can then be disposed over the top of the well 106.

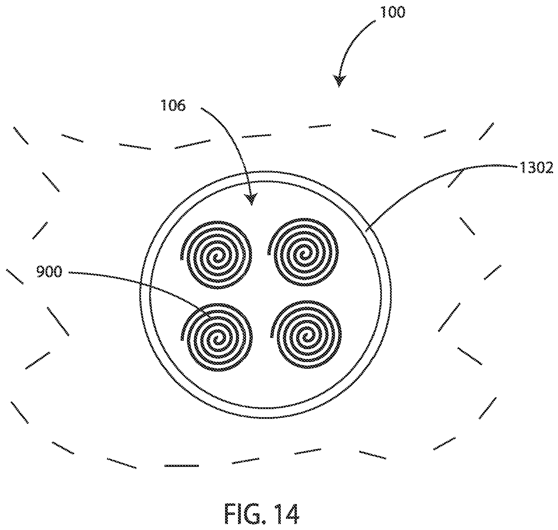

In some embodiments, a plurality of chemical sensor elements 900 can be disposed in the well 106. Referring now to FIG. 14, a plurality of chemical sensor elements 900 disposed in well 106 are shown in accordance with various embodiments herein. It can be appreciated that increasing the number of chemical sensor elements 900 disposed in the well 106 can increase the detection capabilities and sensitivity of chemical sensing of the implantable medical device 100. In some embodiments, all of the chemical sensor elements 900 are substantially identical. However, in some embodiments, some of the chemical sensor elements 900 can be different. For example, some of the chemical sensor elements 900 can include a different sensing composition than the other chemical sensor elements 900.

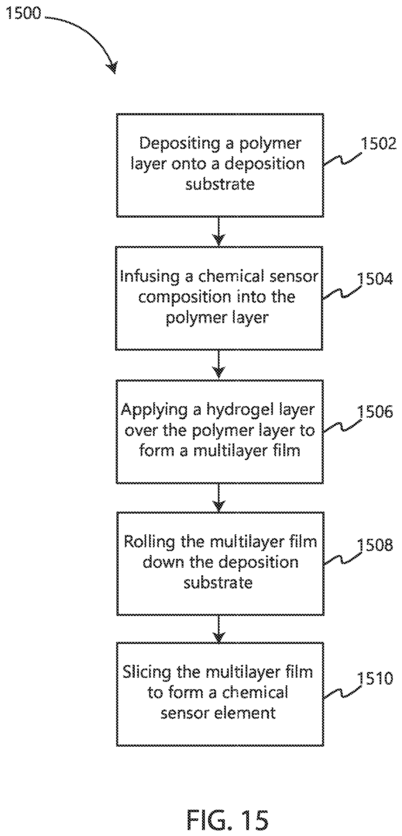

Referring now to FIG. 15, a flow diagram of a method 1500 of making a chemical sensor element is shown in accordance with various embodiments herein. The method 1500 can include an operation of depositing 1502 a polymer layer onto a deposition substrate. In some embodiments, the polymer layer can be deposited using an electrospinning process. However, in other embodiments, the polymer layer can be deposited using a different deposition process including a conventional spraying process (including, but not limited to electrospraying), dip coating, blade coating, print deposition, or the like. The polymer layer can be formed of a hydrophobic polymer. The polymer layer can be porous.

The method 1500 can include an operation of infusing 1504 a chemical sensor composition into the polymer layer. In some embodiments, the chemical sensor composition can be sprayed onto the surface of the polymer layer and then be allowed to infuse there into. In other embodiments, the chemical sensor composition can be applied to the polymer layer using a different technique. As a result, the chemical sensing composition can be dispersed within the polymer layer.

Depending on the technique used to applying the hydrophobic polymer layer, in some embodiments the chemical sensor composition can be mixed in with the hydrophobic polymer composition before it is applied to the mandrel or other substrate.

The method 1500 can include an operation of applying 1506 a hydrogel layer over the polymer layer to form a multilayer film. The hydrogel layer can be applied over the polymer layer using any conventional spraying process or using another deposition process. More generally, the method 1500 can include an operation of applying 1506 a hydrophilic polymer layer over a hydrophobic polymer layer to form a multilayer film.

The method 1500 can also include an operation of rolling 1508 the multilayer film down the deposition substrate. In some embodiments, this operation can be performed last, such as after the multilayer film is sliced 1510. In other embodiments, this operation be performed after the hydrogel layer is applied over the polymer layer but before the multilayer film is sliced 1510. Regardless, the method 1500 can include an operation of slicing 1510 the multilayer film to form a chemical sensor element.

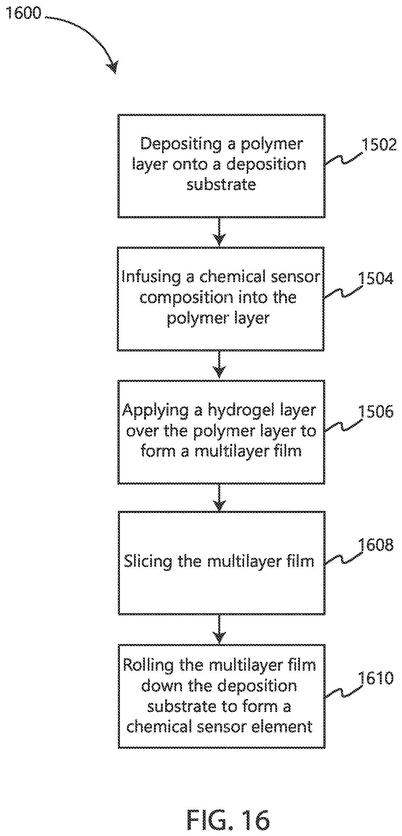

Referring now to FIG. 16, a flow diagram of a method 1600 of making a chemical sensor element is shown in accordance with various embodiments herein. As before, the method 1600 can include operations of depositing 1502 a polymer layer onto a deposition substrate, infusing 1504 a chemical sensor composition into the polymer layer, and applying 1506 a hydrogel layer over the polymer layer to form a multilayer film.

However, method 1600 can include an operation of slicing 1608 the multilayer film followed by an operation of rolling 1610 the multilayer film down the deposition substrate to form a chemical sensor element (instead of in the reverse order). In some embodiments, an operation of modifying the polymer layer (e.g., the layer deposited on the deposition substrate) can also be included so as to allow it to more readily accepts the chemical sensor composition.

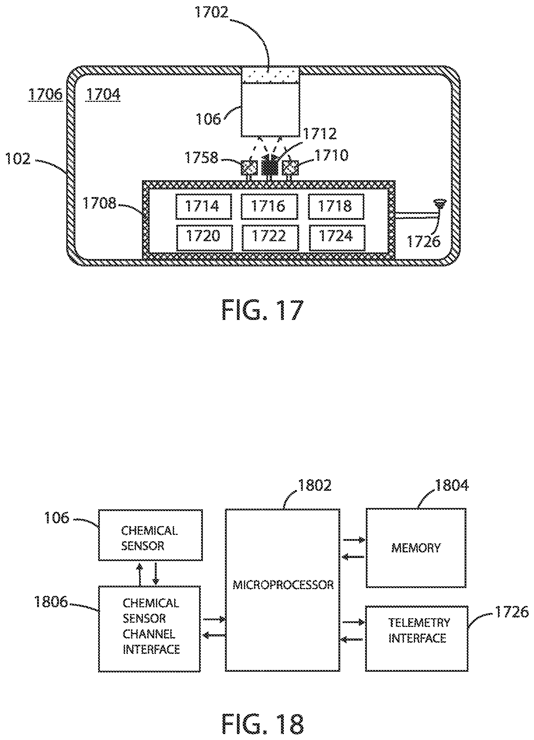

It will be appreciated that implantable medical devices herein can include many other components beyond the chemical sensing element. Referring now to FIG. 17, a schematic view of implantable medical device 100 is shown in accordance with various embodiments herein. The implantable medical device 100 can include implantable housing 102. The implantable housing 102 of the implantable medical device 100 can include various materials such as metals, polymers, ceramics, and the like. In some embodiments, the implantable housing 102 can be a single integrated unit. In other embodiments, the implantable housing 102 can include implantable housing 102 and epoxy header 104, as discussed above. In some embodiments, the implantable housing 102, or one or more portions thereof, can be formed of titanium. In some embodiments, one or more segments of the implantable housing 102 can be hermetically sealed.

The implantable housing 102 can define an interior volume 1704 that in some embodiments is hermetically sealed off from the area 1706 outside of the implantable medical device 100. The implantable medical device 100 can include well 106 which can hold one or more chemical sensor elements 900, as discussed above. The well 106 can be covered by a cover layer or a window 1702.

The window 1702 can be formed from a permeable material, such as an ion permeable polymeric matrix material. Many different materials can be used as the ion permeable polymeric matrix material. In some embodiments, the ion permeable polymeric matrix material can be a hydrogel. In some embodiments, the ion permeable polymeric material can be polyhydroxyethyl methacrylate (polyHEMA) either as a homopolymer or a copolymer including the same. The ion permeable polymeric matrix material(s) can be chosen based on its permeability to one or more of an electrolyte, a protein, a sugar, a hormone, a peptide, an amino acid, or a metabolic product. Specific ion permeable polymeric matrix material are discussed in more detail below. In some embodiments, the window 1702 can be opaque to the passage of light in one or more of the visible, ultraviolet (UV), or near-infrared (NIR) frequency spectrums.

The implantable medical device 100 can further include circuitry 1708. The circuitry 1708 can include various components, such as components 1714, 1716, 1718, 1720, 1722, and 1724. In some embodiments, these components can be integrated and in other embodiments these components can be separate. In some embodiments, the components can include one or more of control circuitry (microprocessor, microcontroller, an ASIC, or the like), memory circuitry (such as random access memory (RAM) and/or read only memory (ROM)), recorder circuitry, telemetry circuitry, chemical sensor interface circuitry, power supply circuitry (which can include one or more batteries), normalization circuitry, chemical sensor control circuitry, and the like. In some embodiments, recorder circuitry can record the data produced by the chemical sensor and record time stamps regarding the same. In some embodiments, the circuitry can be hardwired to execute various functions, while in other embodiments the circuitry can be implemented as instructions executing on a microprocessor or other computation device.

A telemetry interface 1726 can be provided for communicating with external devices such as a programmer, a home-based unit, and/or a mobile unit (e.g., a cellular phone, portable computer, etc.). In some embodiments, the telemetry interface 1726 can be provided for communicating with implanted devices such as a therapy delivery device (e.g. a pacemaker, cardiovertor-defibrillator) or monitoring only device (e.g. an implantable loop recorder). In some embodiments, the circuitry can be implemented remotely, via either near-field, far-field, conducted, intra-body or extracorporeal communication, from instructions executing on any of the external or the implanted devices, etc. In some embodiments, the telemetry interface 1726 can be located within the implantable housing 102. In some embodiments, the telemetry interface 1726 can be located in header 104.

An optical excitation assembly 1712 as well as optical detection assemblies 1710, 1758 can be in electrical communication with the circuitry 1708 within the interior volume 1704. In some embodiments, the control circuitry 1708 is configured to selectively activate the optical excitation 1712 and optical detection assemblies 1710, 1758. The optical excitation assembly 1712 can be configured to cast light onto one or more chemical sensing elements disposed within the well 106. The optical detection assemblies 1710, 1758 are configured to receive light from the one or more chemical sensor elements.

While FIG. 17 shows light rays reflecting and/or absorbing near the bottom of the well / sensing elements, it will be appreciated that this is merely for ease of illustration and that the entire sensing element can be exposed to light from the optical excitation assemblies and therefore contribute to generating a response that can be detected by the optical detection assemblies.

Referring now to FIG. 18, a schematic diagram of components of implantable medical device 100 in accordance with various embodiments herein. It will be appreciated that some embodiments can include additional elements beyond those shown in FIG. 18. In addition, some embodiments may lack some elements shown in FIG. 18. The implantable medical device 100 can gather information through one or more sensing channels. A microprocessor 1802 can communicate with a memory 1804 via a bidirectional data bus. The memory 1804 can include read only memory (ROM) or random access memory (RAM) for program storage and RAM for data storage, or any combination thereof. The implantable medical device 100 can also include one or more chemical sensors 108 and one or more chemical sensor channel interfaces 1806 which can communicate with a port of the microprocessor 1802. The chemical sensor channel interface 1806 can include various components such as analog-to-digital converters for digitizing signal inputs, sensing amplifiers, registers which can be written to by the control circuitry in order to adjust the gain and threshold values for the sensing amplifiers, source drivers, modulators, demodulators, multiplexers, and the like. A telemetry interface 1726 is also provided for communicating with external devices such as a programmer, a home-based unit, and/or a mobile unit (e.g., a cellular phone, portable computer, etc.), implanted devices such as a pace maker, cardiovertor-defibrillator, loop recorder, and the like.

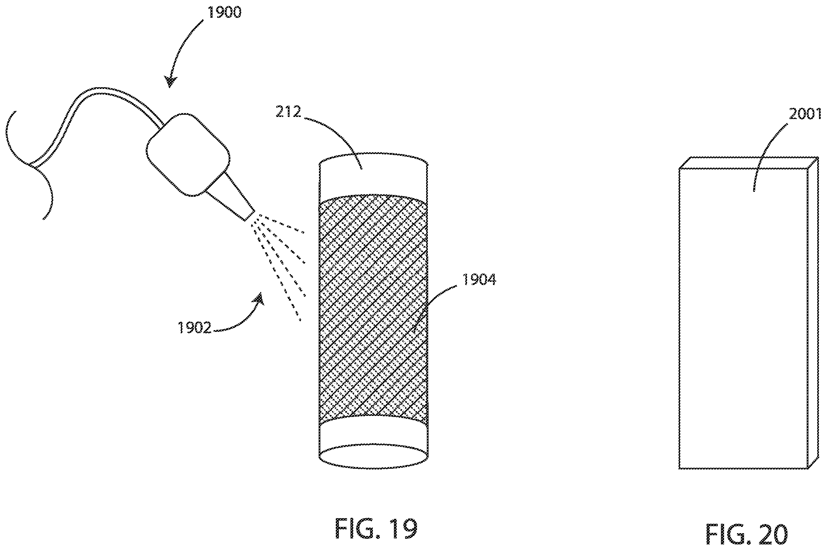

In some embodiments, a hydrophobic layer 1904 (equivalent in function to the fibrous layer 302) can be deposited onto mandrel 212. For example, FIG. 19 shows a hydrophobic layer 1904 being sprayed onto mandrel 212. Sprayer 1900 can spray a hydrophobic polymer composition 1904 onto the mandrel 212 to form the hydrophobic layer 1904. In some embodiments, the spraying process can be performed using any conventional sprayer.

While in some embodiments layers herein may be deposited onto a mandrel, it will be appreciated that, more broadly, layers can be disposed on various deposition substrates (which may not be cylindrical) before later being removed to form a rolled component. It will be appreciated that deposition substrates can come in various shapes and sizes. Referring now to FIG. 20, a deposition substrate is shown in accordance with various embodiments herein. FIG. 20 shows a deposition substrate 2001. In some embodiments, the deposition substrate 2002 can be planar, rectangular, cylindrical, conical, columnar, pyramidal, polygonal, or the like.

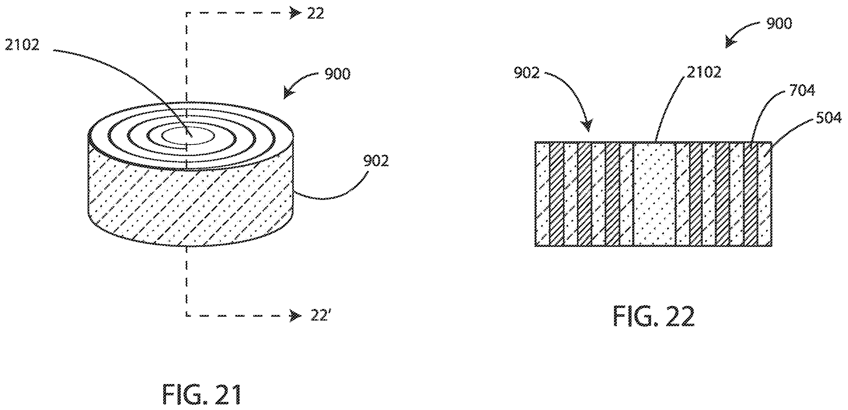

In various embodiments, multiple chemical sensor elements can be disposed within a well space and it can be desirable to let light penetrate throughout the well so that it may illuminate all of the chemical sensor elements disposed therein. This can be accomplished in various ways. For example, in some embodiments, a light pipe can be placed in the center of a chemical sensor element. Referring now to FIG. 21, chemical sensor element 900 including a light pipe 2102 is shown in accordance with various embodiments herein. The chemical sensor element 900 can include a rolled multilayer film 902. The rolled multilayer film 902 can include a light pipe 2102 at the center of the roll. The light pipe 2102 can serve to convey light from one side of the chemical sensor element 900 to the other without substantial interference. Thus, in a case where a well is filled with a plurality of chemical sensor elements, some light can be passed through a first chemical sensor element so as to allow the light to illuminate other chemical sensor elements within the well that may otherwise be shielded by the first chemical sensor element. In some embodiments, the light pipe 2102 can be replaced by another component to convey light without substantial interference such as a fiber optic component, an empty chamber, or the like.

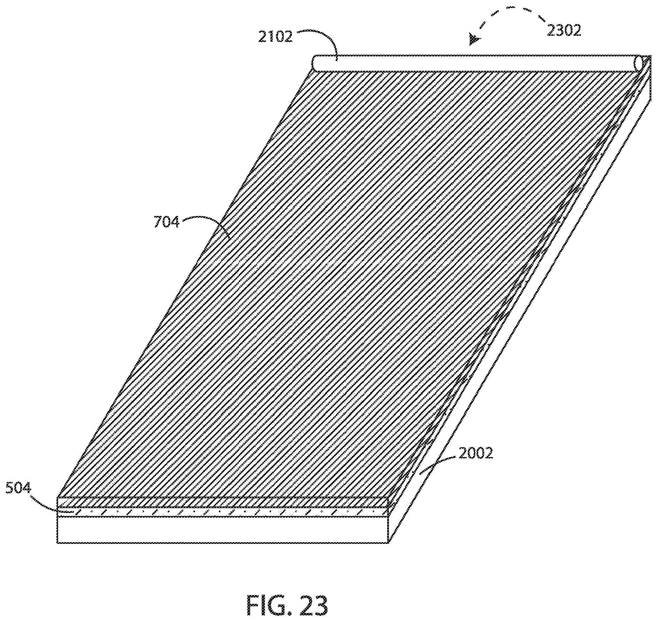

Referring now to FIG. 22, a cross-sectional view of a chemical sensor element taken along line 22-22' of FIG. 21 is shown in accordance with various embodiments herein showing the light pipe 2102 disposed at the center of the rolled multilayer film 902. In some embodiments, light pipe 2102 can be placed in the center of the rolled multilayer film 902 (e.g., after rolling has occurred). In other embodiments, referring now to FIG. 23, the light pipe 2102 can be placed on top of the fibrous layer with infused sensing composition 504 and the hydrogel layer 704 and then rolled up inside.

### Methods

Many different methods are contemplated herein, including, but not limited to, methods of making, methods of using, and the like. Aspects of system/device operation described elsewhere herein can be performed as operations of one or more methods in accordance with various embodiments herein.

In an embodiment, a method of making a chemical sensor element is included, the method depositing a polymer layer onto a deposition substrate, infusing a chemical sensor composition into the polymer layer, applying a hydrogel layer over the polymer layer to form a multilayer film, rolling the multilayer film down the deposition substrate, and slicing the multilayer film to form the chemical sensor element.

In an embodiment of the method, depositing the polymer layer onto the deposition substrate further comprises electrospinning a fiber layer onto the deposition substrate. In other embodiments of the method, the polymer layer can be sprayed on the deposition substrate, painted/brushed on the deposition substrate, or any other appropriate method to apply onto the deposition substrate.

In an embodiment of the method, the deposition substrate is substantially cylindrical. In other embodiments of the method, the deposition substrate can be rectangular, conical, columnar, pyramidal, or any other suitable shape.

In an embodiment of the method, applying the hydrogel layer over the polymer layer to form the multilayer film further comprises spraying a hydrogel composition onto the fiber layer. In other embodiments of the method, the hydrogel layer can be painted/brushed on the polymer layer, or any other appropriate method to apply onto the polymer layer.

In an embodiment of the method, the operation of slicing occurs after the operation of rolling. It is considered that the operation of slicing occurring after the operation of rolling can generate rolled multilayer films that are uniform in size and shape. In other embodiments of the method, the operation of slicing occurs before the operation of rolling. The operation of slicing occurring before the operation of rolling can allow for rolled multilayer films to be created that are more spiral in nature.

In an embodiment, the method can further include placing the multilayer film into a well after the operations of rolling and slicing.

In an embodiment, the method can further include applying a second hydrogel layer over the multilayer film after the operations of rolling and slicing. While the second hydrogel layer can be applied over the multilayer film for a variety of reasons, it is contemplated the hydrogel layer can be applied to touch up any the hydrogel layer that may have been removed during the rolling or slicing of the rolled multilayer film. It is further contemplated the hydrogel layer can be applied to provide further structural support to the rolled multilayer film and/or protect the rolled multilayer film during its placement into the well or while in use.

In an embodiment, the method can further include placing a light pipe over the multilayer film prior to rolling. It is considered that the light pipe can allow ions to permeate into a center of the rolled multilayer film thereby increasing the detection capabilities of the rolled multilayer film.

### Chemical Sensing Composition

Various embodiments herein include a chemical sensing composition. Further details about the chemical sensing composition are provided as follows. However, it will be appreciated that this is merely provided by way of example and that further variations are contemplated herein.

As referenced above, the chemical sensing composition of the chemical sensor element can be formed of a lipophilic indicator dye. In some embodiments, the chemical sensing composition can be formed from a lipophilic fluorescent indicator dye. In other embodiments, the chemical composition can be formed from a lipophilic colorimetric indicator dye. Suitable lipophilic indicator dyes can include, but are not limited to, ion selective sensors such as ionophores or fluorophores.

In some embodiments, ionophores herein can include, but not be limited to, sodium specific ionophores, potassium specific ionophores, calcium specific ionophores, magnesium specific ionophores, and lithium specific ionophores. In some embodiments, fluorophores can include, but not be limited to, lithium specific fluorophores, sodium specific fluorophores, and potassium specific fluorophores.

Chemical sensing compositions herein can include components (or response elements) that are configured for a colorimetric response, a photoluminescent response, or another optical sensing modality.

Colorimetric response elements herein can be specific for a particular chemical analyte. Colorimetric response elements can include an element that changes color based on binding with or otherwise complexing with a specific chemical analyte. In some embodiments, a colorimetric response element can include a complexing moiety and a colorimetric moiety. Those moieties can be a part of a single chemical compound (as an example a non-carrier based system) or they can be separated on two or more different chemical compounds (as an example a carrier based system). The colorimetric moiety can exhibit differential light absorbance on binding of the complexing moiety to an analyte.

Photoluminescent response elements herein can be specific for a particular chemical analyte. Photoluminescent response elements herein can include an element that absorbs and emits light through a photoluminescent process, wherein the intensity and/or wavelength of the emission is impacted based on binding with or otherwise complexing with a specific chemical analyte. In some embodiments, a photoluminescent response element can include a complexing moiety and a fluorescing moiety. Those moieties can be a part of a single chemical compound (as an example a non-carrier based system) or they can be separated on two or more different chemical compounds (as an example a carrier based system). In some embodiments, the fluorescing moiety can exhibit different fluorescent intensity and/or emission wavelength based upon binding of the complexing moiety to an analyte.

Some chemistries may not require a separate compound to both complex an analyte of interest and produce an optical response. By way of example, in some embodiments, the response element can include a non-carrier optical moiety or material wherein selective complexation with the analyte of interest directly produces either a colorimetric or fluorescent response. As an example, a fluoroionophore can be used and is a compound including both a fluorescent moiety and an ion complexing moiety. As merely one example, (6,7-[2.2.2]-cryptando-3-[2"-(5"-carboethoxy)thiophenyl]coumarin, a potassium ion selective fluoroionophore, can be used (and in some cases covalently attached to polymeric matrix or membrane) to produce a fluorescence-based K⁺ non-carrier response element.

An exemplary class of fluoroionophores are the coumarocryptands. Coumarocryptands can include lithium specific fluoroionophores, sodium specific fluoroionophores, and potassium specific fluoroionophores. For example, lithium specific fluoroionophores can include (6,7-[2.1.1]-cryptando-3-[2"-(5"-carboethoxy)furyl]coumarin. Sodium specific fluoroionophores can include (6,7-[2.2.1]-cryptando-3-[2"-(5"-carboethoxy)furyl]coumarin. Potassium specific fluoroionophores can include (6,7-[2.2.2]-cryptando-3-[2"-(5"-carboethoxy)furyl]coumarin and (6,7-[2.2.2]-cryptando-3-[2"-(5"-carboethoxy)thiophenyl]coumarin.

Suitable fluoroionophores include the coumarocryptands taught in U.S. Pat. No. 5,958,782. Such fluorescent ionophoric compounds can be excited with GaN blue light emitting diodes (LEDs) emitting light at or about 400 nm. These fluorescent ionophoric compounds have ion concentration dependent emission that can be detected in the wavelength range of about 450 nm to about 470 nm.

Some chemistries can rely upon a separate complexing entity (e.g., a separate chemical compound). As an example, carrier based response elements can include a compound, in some cases referred to as an ionophore, that complexes with and serves to carry the analyte of interest. In some embodiments, carrier based response elements include a lipophilic ionophore, and a lipophilic fluorescent or colorimetric indicator dye, called a chromoionophore. In some cases the chromoionophore and the ionophore can be dispersed in, and/or covalently attached to, a hydrophobic organic polymeric matrix. The ionophore can be capable of reversibly binding ions of interest. The chromoionophore can be a proton selective dye. In operation, analytes of interest are reversibly sequestered by the ionophores within the organic polymer matrix. To maintain charge neutrality within the polymer matrix, protons are then released from the chromoionophore, giving rise to a color or fluorescence change. As just one specific example, a carrier based response element can include potassium ionophore III, chromoionophore I, and potassium tetrakis(4-chlorophenyl)borate dispersed in a polymer matrix made from polyvinylchloride and bis(2-ethylhexyl)sebacate surfactant to produce a colorimetric K⁺ sensing element.

Both non-carrier based response elements and carrier-based response elements can include complexing moieties. Suitable complexing moieties can include cryptands, crown ethers, bis-crown ethers, calixarenes, noncyclic amides, and hemispherand moieties as well as ion selective antibiotics such as monensin, valinomycin and nigericin derivatives.

Those of skill in the art can recognize which cryptand and crown ether moieties are useful in complexing particular cations, although reference can be made to, for example, Lehn and Sauvage, "[2]-Cryptates: Stability and Selectivity of Alkali and Alkaline-Earth Macrocyclic Complexes," J. Am. Chem. Soc, 97, 6700-07 (1975), for further information on this topic. Those skilled in the art can recognize which bis-crown ether, calixarene, noncyclic amides, hemispherand, and antibiotic moieties are useful in complexing particular cations, although reference can be made to, for example, Buhlmann et al., "Carrier-Based Ion-Selective Electrodes and Bulk Optodes. 2. Ionophores for Potentiometric and Optical Sensors," Chem. Rev. 98, 1593-1687 (1998), for further information on this topic.

By way of example cryptands can include a structure referred to as a cryptand cage. For cryptand cages, the size of the cage is defined by the oxygen and nitrogen atoms and the size makes cryptand cages quite selective for cations with a similar diameter. For example a [2.2.2] cryptand cage is quite selective for cations such as K⁺, Pb⁺², Sr⁺², and Ba⁺². A [2.2.1] cryptand cage is quite selective for cations such as Na⁺ and Ca⁺². Finally, a [2.1.1] cryptand cage is quite selective for cations such as Li⁺ and Mg⁺². The size selectivity of cryptand cages can aid in the sensitivity of chemical sensing. When these cryptand cages are incorporated into physiologic sensing systems heavier metals such as Pb⁺² and Ba⁺² are unlikely to be present in concentrations which interfere with the analysis of ions of broader physiological interest such as Na⁺ and K⁺.

Further aspects of chemical sensor compositions are described in U.S. Pat. Nos. 7,809,441 and 8,126,554.

### Hydrophilic Polymers

Various embodiments herein include a hydrophilic polymer. Further details about the hydrophilic polymer are provided as follows. However, it will be appreciated that this is merely provided by way of example and that further variations are contemplated herein.

As referenced above, the hydrophilic polymer layer of the chemical sensor element can be formed of a permeable material. In some embodiments, the hydrophilic polymer layer can be formed from an ion-permeable polymeric matrix material. Suitable polymers for use as the ion-permeable polymeric matrix material can include, but are not limited to polymers forming a hydrogel. Hydrogels herein can include homopolymeric hydrogels, copolymeric hydrogels, and multipolymer interpenetrating polymeric hydrogels. Hydrogels herein can specifically include nonionic hydrogels.

In some embodiments, hydrogels herein can be prepared from polymerization of various monomers or macromers including one or more of poly 2-hydroxyethl methacrylate (polyHEMA), 2-hydroxypropyl methacrylate (HPMA), acrylamide, acrylic acid, N-isopropylacrylamide (NIPAm), methoxyl polyethylene glycol monocrylate (PEGMA), and the like. In some embodiments, polymers can include, but are not limited to polyhydroxyethyl methacrylate (HEMA), cellulose, polyvinyl alcohol, polyacrylate, dextran, polyacrylamides, polyhydroxyalkyl acrylates, polyvinyl pyrrolidones, and mixtures and copolymers thereof.

### Hydrophobic Polymers

Various embodiments herein include a hydrophobic polymer. Further details about the hydrophobic polymer are provided as follows. However, it will be appreciated that this is merely provided by way of example and that further variations are contemplated herein.

As referenced above, the hydrophobic polymer layer of the chemical sensor element can be formed of a biocompatible thermoplastic polymer. Suitable polymers for use as biocompatible thermoplastic polymers can include, but are not limited to homopolymeric thermoplastics, copolymeric thermoplastics, multipolymer interpenetrating polymeric thermoplastics, and any polymers capable of being electrospun.

In some embodiments, biocompatible thermoplastics herein can include, but are limited to polyvinylchloride (PVC), polysulfone (PS), polytetrafluorethylene (PTFE), polyethylene (PE), polypropylene (PP), polyethersulfone (PES), polyurethane (PU), polyetherimide (PEI), polycarbonate (PC), polyetheretherketone (PEEK), cellulose, and the like.

In some embodiments the biocompatible thermoplastic can include biocompatible thermoplastic polymers infused with plasticizers to increase the flexibility and plasticity of the biocompatible thermoplastic while decreasing its viscosity and friction. Plasticizers can include one or more of bis(2-ethylhexyl) phthalate (DEHP), diisooctyl phthalate (DIOP), acetyl tributyl citrate (ATBC), acetyl trihexyl citrate (ATHC), butyryl trihexyl citrate (BTHC), trimethyl citrate (TMC), and the like.

In some embodiments, the hydrophobic polymers can exist in the form of fibers, such as would be the result of an electrospraying process. In some embodiments, the fibers can specifically include polyvinyl chloride and a plasticizer. The fibers can be of various diameters. In some embodiments, the fibers can have an average diameter of greater than or equal to 100 nm, 200 nm, 300 nm, 400 nm, 500 nm, or 600 nm. In some embodiments, the average diameter can be less than or equal to 2000 nm, 1720 nm, 1440 nm, 1160 nm, 880 nm, or 600 nm. In some embodiments, the average diameter can fall within a range of 100 nm to 2000 nm, or 200 nm to 1720 nm, or 300 nm to 1440 nm, or 400 nm to 1160 nm, or 500 nm to 880 nm, or can be about 600 nm.

### Optical Excitation and Detection Assemblies

In some embodiments , optical excitation assemblies herein can include solid state light sources such as GaAs, GaAlAs, GaAlAsP, GaAIP, GaAsp, Gap, GaN, InGaAIP, InGaN, ZnSe, or SiC light emitting diodes or laser diodes that excite the chemical sensor element(s) 900 at or near the wavelength of maximum absorption for a time sufficient to emit a return signal. However, it will be understood that in some embodiments the wavelength of maximum absorption reflection varies as a function of concentration in the colorimetric sensor.

In some embodiments, the optical excitation assemblies can include other light emitting components including incandescent components. In some embodiments, the optical excitation assemblies can include a wave guide. The optical excitation assembly can also include one or more bandpass filters, high pass filter, low pass filter, antireflection elements, and/or focusing optics.

In some embodiments, the optical excitation assembly can include a plurality of LEDs with bandpass filters, each of the LED-filter combinations emitting at a different center frequency. According to various embodiments, the LEDs can operate at different center-frequencies, sequentially turning on and off during a measurement, illuminating the chemical sensor element. As multiple different center-frequency measurements are made sequentially, a single unfiltered detector can be used in some embodiments. However, in some embodiments, a polychromatic source can be used with multiple detectors that are each bandpass filtered to a particular center frequency.

The chemical sensor element can include the rolled multilayer film having embedded therein various types of ion selective sensors. Physiological analytes of interest can diffuse into and out of the chemical sensor element and bind with an ion selective sensor to result in a fluorimetric or colorimetric response. Reference analytes can similarly diffuse into and out of the chemical sensor element and serve as a control sample. Exemplary ion selective sensors are described more fully below.

The optical detection assemblies can be configured to receive light from the chemical sensor element. In an embodiment, the optical detection assemblies can include a component to receive light. By way of example, in some embodiments, the optical detection assemblies can include a charge-coupled device (CCD). In other embodiments, the optical detection assemblies can include a photodiode, a junction field effect transistor (JFET) type optical sensor, or a complementary metal-oxide semiconductor (CMOS) type optical sensor. In some embodiments, the optical detection assemblies can include an array of optical sensing components. In some embodiments, the optical detection assemblies can include a waveguide.

The optical detection assemblies can also include one or more bandpass filters and/or focusing optics. In some embodiments, the optical detection assemblies can include one or more photodiode detectors, each with an optical bandpass filter tuned to a specific wavelength range.

The optical excitation and detection assemblies respectively, can be integrated using bifurcated fiber-optics that direct excitation light from a light source to one or more chemical sensor elements, or simultaneously to chemical sensor element(s) and a reference channel. Return fibers can direct emission signals from the chemical sensor element(s) and the reference channels to one or more optical detector assemblies for analysis by a processor, such as a microprocessor. In some embodiments, the optical excitation and detection assemblies are integrated using a beam splitter assembly and focusing optical lenses that direct excitation light from a light source to the sensing element and direct emitted or reflected light from the sensing element to an optical detector for analysis by a processor.

It should be noted that, as used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

All publications and patent applications in this specification are indicative of the level of ordinary skill in the art to which this invention pertains.

As used herein, the recitation of numerical ranges by endpoints shall include all numbers subsumed within that range (e.g., 2 to 8 includes 2.1, 2.8, 5.3, 7, etc.).

The headings used herein are provided for consistency with suggestions under 37 CFR 1.77 or otherwise to provide organizational cues. These headings shall not be viewed to limit or characterize the invention(s) set out in any claims that may issue from this disclosure. As an example, although the headings refer to a "Field," such claims should not be limited by the language chosen under this heading to describe the so-called technical field. Further, a description of a technology in the "Background" is not an admission that technology is prior art to any invention(s) in this disclosure. Neither is the "Summary" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques.

## Claims

1. A method of making a chemical sensor element (900) comprising:
depositing a polymer layer onto a deposition substrate;
infusing a chemical sensor composition into the polymer layer;
applying a hydrogel layer (1202) over the polymer layer to form a multilayer film (902);
**characterised by** said method further comprising rolling the multilayer film down the deposition substrate; and
slicing the multilayer film to form the chemical sensor element.

2. The method of any of claims 1, wherein depositing the polymer layer onto the deposition substrate further comprises electrospinning a fiber layer onto the deposition substrate.

3. The method of any of claims 1-2, wherein the fiber layer comprises fibers, the fibers comprising polyvinyl chloride and a plasticizer.

4. The method of any of claims 1-3, wherein applying the hydrogel layer over the polymer layer to form the multilayer film further comprises spraying a hydrogel composition onto the polymer layer.

5. The method of any of claims 1-4, further comprising applying a second hydrogel layer over the multilayer film after the operations of rolling and slicing.

6. The method of any of claims 1-5, further comprising placing a light pipe (2102) over the multilayer film prior to rolling.

7. A chemical sensor element formed according to the method of claim 1.

## Patentansprüche

1. Verfahren zur Herstellung eines chemischen Sensorelements (900), umfassend:
Abscheiden einer Polymerschicht auf ein Abscheidungssubstrat;
Einbringen einer chemischen Sensorzusammensetzung in die Polymerschicht;
Aufbringen einer Hydrogelschicht (1202) über der Polymerschicht, um einen mehrschichtigen Film (902) zu bilden;
**dadurch gekennzeichnet, dass** das Verfahren des Weiteren das Abrollen des mehrschichtigen Films auf dem Abscheidungssubstrat umfasst; und
Schneiden des mehrschichtigen Films, um das chemische Sensorelement zu bilden.

2. Verfahren nach einem der Ansprüche 1,
wobei das Abscheiden der Polymerschicht auf das Abscheidungssubstrat des Weiteren das Elektrospinnen einer Faserschicht auf das Abscheidungssubstrat umfasst.

3. Verfahren nach einem der Ansprüche 1 bis 2,
wobei die Faserschicht Fasern umfasst, wobei die Fasern Polyvinylchlorid und einen Weichmacher umfassen.

4. Verfahren nach einem der Ansprüche 1 bis 3,
wobei das Aufbringen der Hydrogelschicht über der Polymerschicht, um einen mehrschichtigen Film zu bilden, des Weiteren das Sprühen einer Hydrogelzusammensetzung auf die Polymerschicht umfasst.

5. Verfahren nach einem der Ansprüche 1 bis 4,
des Weiteren umfassend das Aufbringen einer zweiten Hydrogelschicht über dem mehrschichtigen Film nach den Vorgängen des Abrollens und Schneidens.

6. Verfahren nach einem der Ansprüche 1 bis 5, des Weiteren umfassend das Platzieren einer Lichtröhre (2102) über dem mehrschichtigen Film vor dem Abrollen.

7. Chemisches Sensorelement, das gemäß dem Verfahren nach Anspruch 1 gebildet wird.

## Revendications

1. Procédé de fabrication d'un élément de capteur chimique (900) comprenant :
le dépôt d'une couche de polymère sur un substrat de dépôt ;
l'infusion d'une composition de capteur chimique à l'intérieur de la couche de polymère ; et
l'application d'une couche d'hydrogel (1202) au-dessus de la couche de polymère afin de former un film multicouche (902) ;
**caractérisé en ce que** ledit procédé comprend en outre l'enroulement du film multicouche sur le substrat de dépôt ; et
le tranchage du film multicouche afin de former l'élément de capteur chimique.

2. Procédé selon la revendication 1, dans lequel le dépôt de la couche de polymère sur le substrat de dépôt comprend en outre le filage électrostatique d'une couche de fibres sur le substrat de dépôt.

3. Procédé selon l'une quelconque des revendications 1 et 2, dans lequel la couche de fibres comprend des fibres, les fibres comprenant du chlorure de polyvinyle et un plastifiant.

4. Procédé selon l'une quelconque des revendications 1 à 3, dans lequel l'application de la couche d'hydrogel au-dessus de la couche de polymère afin de former le film multicouche comprend en outre la pulvérisation d'une composition d'hydrogel sur la couche de polymère.

5. Procédé selon l'une quelconque des revendications 1 à 4, comprenant en outre l'application d'une seconde couche d'hydrogel au-dessus du film multicouche après les opérations d'enroulement et de tranchage.

6. Procédé selon l'une quelconque des revendications 1 à 5, comprenant en outre le placement d'un conduit de lumière (2102) au-dessus du film multicouche avant l'enroulement.

7. Élément de capteur chimique formé selon le procédé de la revendication 1.
